# EUROPEAN PATENT APPLICATION

(11) **EP 1 269 840 A1**
(43) Date of publication of application: **02.01.2003**
(21) Application number: 01908246.0
(22) Date of filing: 02.03.2001
(51) Int. Cl.: A01N 1/02, A61F 2/06, A61F 2/10, A61F 2/14, A61L 27/38, A61L 27/60

(54) **METHOD OF PRESERVING TISSUE EQUIVALENT AND TISSUE EQUIVALENT PRESERVED IN FROZEN STATE**

(30) Priority: 24.03.2000 JP 2000085235
(71) Applicant: MENICON CO., LTD., Nagoya-shi, Aichi 460-0006 (JP)
(72) Inventor: YAMAMOTO, Naoka, c/o MENICON CO., LTD, Kasugai-shi, Aichi 487-0032 (JP); NOMURA, Masayo, c/o MENICON CO., LTD, Kasugai-shi, Aichi 487-0032 (JP); MORIYAMA, Takeshi, c/o MENICON CO., LTD, Kasugai-shi, Aichi 487-0032 (JP)
(74) Representative: Leson, Thomas Johannes Alois, Dipl.-Ing.
(86) International application number: JP0101619
(87) International publication number: WO01070021

(57) **Abstract**

A method for cryopreservation of a tissue equivalent whereby the viability of frozen cells and the biological activity of thawed cells are improved and the steps are simplified; and a cryopreserved tissue equivalent obtained by the method. Cells suspended in a cryopreserving solution are inoculated into a matrix and then frozen before the cells adhere to the matrix.

## Description

### TECHNICAL FIELD

The present invention relates to a method for cryopreservation of a tissue equivalent and a cryopreserved tissue equivalent. In more detail, the present invention relates to a method for cryopreservation of a tissue equivalent which comprises a step of suspending cells in a cryopreserving solution, a step of inoculating the cells on a matrix and a step of freezing the thus obtained tissue equivalent before the cells adhere to the matrix, and a cryopreserved tissue equivalent obtained by the method.

### BACKGROUND ART

As the conventional general method for preserving a tissue equivalent, there is taken a method which comprises suspending cells in a medium, inoculating the cells on a matrix, culturing the cells to adhere to the matrix, and soaking it in a cryopreserving solution for a period of time to equilibrate and, thereafter, cooling gradually and freezing.

However, the conventional method has problems that the viability of cells is very low and, further, that a very precise and expensive freezing apparatus such a program freezer is required for adjusting a cooling constant. In addition, the conventional method needs a step of preculturing cells for adhesion of the cells to a matrix (from a few hours to overnight) and a step of washing the cells with a cryopreserving solution before freezing and, further, also has a problem that it takes a longer period of time to permeate and to equilibrate the cryopreserving solution into the cells.

In particular, in an artificial skin, a method for cryopreserving sheet-like epithelial cells is disclosed in Japanese Examined Patent Publication No. 2722134 and a method for immobilizing a cultured skin utilizing a sol-gel method to preserve and transport is disclosed in Japanese Unexamined Patent Publication No. 23968/1996, respectively.

In addition, in Japanese Unexamined Patent Publication No. 505032/1997, there is described a cryopreservation method for a tissue equivalent which comprises soaking the tissue equivalent in a cryoprotectant solution and stirring it to permeate sufficiently the cryoprotectant solution into the tissue equivalent and, thereafter, freezing the tissue equivalent. This method enabled the tissue equivalent to be cryopreserved without damaging the structural integrity while maintaining the cell viability even in a case where the tissue equivalent is comparatively thick and not uniform. However, the method does not comply with simplification of a cryopreservation step in that it takes a time to permeate the cryoprotectant solution.

Furthermore, a cryopreservation method and a thrawing method of a plant cell is described in Japanese Unexamined Patent Publication No. 87102/1997, a cryopreservation method of an artificial liver is described in Japanese Unexamined Patent Publication No. 56897/1978, Japanese Unexamined Patent Publication No. 71755/1990 and Japanese Unexamined Patent Publication No. 506687/1999, and a cryopreservation method of a cornea tissue equivalent is described in U.S.P. 5,374,515, respectively. However, these methods do not sufficiently comply with the high viability and the high biological activity (for example, (a rate of) the proliferating activity, the substance producing ability and the like) of the cells after thawing of the cryopreserved tissue equivalent and simplification of a cryopreserving step.

Accordingly, an object of the present invention is to provide a cryopreservation method of a tissue equivalent, having the improved viability of the cryopreserved cells, the improved biological activity of the thawed cells and the simplified step. A further object of the present invention is to provide a cryopreserved tissue equivalent obtained by the method.

### DISCLOSURE OF INVENTION

As the result of diligent studies in order to solve the above-mentioned problems, the inventors have found that a tissue equivalent can be preserved while maintaining the high viability and the high biological activity of cells, by inoculating the cells suspended in a cryopreserving solution on a matrix, and freezing the resulting tissue equivalent before the cells adhere to the matrix, and the present invention has been accomplished.

That is, the present invention relates to:
a method comprising a step of suspending cells in a cryopreserving solution, a step of inoculating the cells on a matrix, and a step of freezing the thus obtained tissue equivalent before the cells adhere to the matrix, the above method, wherein the cell is one or more selected from the group consisting of fibroblast, epidermal cell, vascular endothelial cell, Langerhans' cell, melanocyte, adipocyte, hair matrix cell, hair papilla cell, smooth muscular cell, hepatocyte, keratocyte, corneal epithelial cell and corneal endothelial cell which are derived from a mammal, and
a cryopreserved tissue equivalent obtained by a method comprising a step of suspending cells in a cryopreserving solution, a step of inoculating the cells on a matrix, and a step of freezing the thus obtained tissue equivalent before the cells adhere to the matrix.

### BEST MODE FOR CARRYING OUT THE INVENTION

"Cells" used in the present invention are finally utilized as an artificial skin, an artificial liver and an artificial cornea. More particularly, in the case of the artificial skin, one or more selected from the group consisting of fibroblast, epidermal cell, Langerhans' cell, melanocyte, adipocyte, hair matrix cell and hair papilla cell which are derived from a mammal are preferable. In the case of the artificial blood vessel, one or more selected from the group consisting of vascular endothelial cell, smooth muscular cell and fibroblast are preferable. In the case of the artificial liver, hepatocyte is preferable. In the case of the artificial cornea, one or more selected from the group consisting of keratocyte, corneal epithelial cell and corneal endothelial cell are preferable.

The term "suspend" in the present invention means an operation by which cells in a solution is dispersed.

In addition, the term "inoculate" in the present invention means to implant cells suspended in a medium into a medium in a culture vessel or on a matrix.

Further, the term "freezing" in the present invention means a step of freezing and preserving a tissue equivalent as soon as it is made by inoculating cells on a matrix, without culturing. Since the viability of cells is very low when the cells are frozen after the cells adhere to the matrix by preculturing, it is preferable that the cells are frozen after the cells are inoculated and before the cells adhere to the matrix. At this time, even when the cells do not adhere to the matrix, the loss of the cells by washing after thawing is little. Here, the adhering refers to the state where cells are not easily freed from a matrix when a physical force is applied. Further, it is more preferable that the cells are frozen during the time when the cells can be easily freed after cells are inoculated. Here, the freeing refers to the state where cells are easily departed from a matrix and can be floated in a solution. Therefore, more particularly, cells must be cooled to 4 to 10°C at such a rate that cells do not adhere to the matrix. When the cooling rate is low, the cells adhere to the matrix and, if a tissue equivalent is frozen in this state, the viability of the cells are lowered. The cooling rate at this time varies depending on a kind of cells, a kind of a cryopreseving solution, the temperature of a tissue equivalent when cooling is initiated and the like. For example, in the case of the artificial skin in which fibroblasts are inoculated in a collagen sponge having a thickness of 2 to 3 mm, it is preferable that the cells are cooled to 4°C within 3 hours when the cells are inoculated at 4°C to normal temperature.

The freezing method in the present invention is not particularly limited but a slow freezing method, a rapid freezing method and a vitrification method are preferable.

Herein, "slow freezing method" is a method for gradually growing an ice crystal formed artificially outside a cell using a cryopreserving solution with a cryoprotectant added, by cooling at a mild and suitable cooling rate, dehydrating and concentrating the interior of the cell at a mild rate and, thereafter, cooling rapidly to prevent intracellular crystallization and, thereby, the high viability after preservation and thawing is obtained (Jpn. J. Emb. Trans., vol.18 No.1, "Vitrificating preservation of in vitro fertilization-derived bovine embryo", Livestock Improvement Association of Japan, Inc., Animal Biotechnology Center). Regarding the cooling rate, the cell is cooled to 4°C at such a rate that the cell does not adhere to the matrix as described the above and, thereafter, the cell is cooled to at least -20°C or lower at a rate of preferably -0.1°C/min. to -10°C/min., more preferably -0.2 to -5°C/min.

"Rapid freezing method" is a method for rapidly cooling and freezing a tissue equivalent (or cells) to a freezing point or lower in a ultra-deep freezer using the cryopreserving solution with the cryoprotectant added. Regarding the cooling rate, the tissue equivalent is cooled to 4°C at such a rate that the cells do not adhere to the matrix as described the above and, thereafter, the tissue equivalent is cooled at a rate of preferably -10°C/min. to -30°C/min., more preferably -10 to -20°C/min. When the rate is greater than -30°C/min., there is a possibility that intracellular crystallization occurs and the cell is broken.

"Vitrification method" is a method for floating cells in a solution with a high concentration of a cryoprotectant added (referred to as a vitrificating solution; normally, the cell-permeable and the non cell-permeable solutions are used together) and, thereafter, rapidly cooling the cell to freeze, for example, by placing into liquid nitrogen. This utilizes phenomenon in which, when a high concentrated aqueous solution is cooled at a high rate, it becomes the state of vitrification without an ice crystal nucleus ("Method of Culturing Germ Cell", Gakujutsushuppan Center, 1993; Study Journal 21 (9), authored by Manabu Shimizu, 1998). According to this method, the tissue equivalent is frozen in a few seconds.

A preserving temperature of a tissue equivalent in the present invention is preferably -20°C to -196°C, more preferably -80°C to -196°C in all of the aforementioned three methods in order to maintain the high biological activity for a longer period of time. When the temperature is higher than -20°C, the viability of cells is lowered in case of preservation for a longer period of time (a few months or longer). Therefore, in case of the preservation for a shorter period of time, -20°C is sufficient. However, in case of preservation for a longer period of time, it becomes necessary to preserve at a lower temperature. The tissue equivalent can be preserved for 1 to 2 years at -80°C and the tissue equivalent can be preserved hemi-permanently at a temperature of -120°C or lower (nitrogen gas) or -196°C (liquid nitrogen).

"Cryopreserving solution" used in the present invention means a solution containing at least one cryoprotectant having the effect of preventing damage of a cell due to freezing.

In the case of the slow freezing method and the rapid freezing method, it is preferable that a cryoprotectant is at least one selected from the group consisting of glycerol, dimethyl sulfoxide (DMSO), propylene glycol, ethylene glycol, sucrose, carboxymethylcellulose salt, carboxymethylcellulose (CMC), monosaccharide and disaccharide (trehalose and the like). As a solvent, an aqueous solution is used. In particular, a physiological salt solution and the like are preferable. The physiological salt solution means a salt solution having a pH and osmotic pressure suitable for cell surviving and includes a physiological sodium chloride (0.9% aqueous solution), a salt solution in which a few main ions such as K⁺ and Ca²⁺ are supplemented to physiological saline, and various cell medium. More particularly, there are DMEM (Dulbecco's Modified Eagle Medium), phosphate buffer, Ringer's solution, Ringer-Locke's solution, Tyrode's solution, Earle's solution, Hanks' solution, Locke's solution, Eagle's minimum essential medium, Ham's synthetic medium F12, Green's medium, Leibovitz's L-15 medium, Cheese essential medium, modified Eagle's medium, Waymouth's medium, Kreb's medium, as well as serum-free medium MCDB153, MCDB151, MCDB104, MCDB131, MCDB402, MCDB201, MCDB302, MCDB105 and MCDB110.

In the case of the vitrification method, as a cryoprotectant, a non cell-permeable vitrificating agent and/or a cell-permeable vitrificating agent are used. As the non cell-permeable vitrificating agent, polyethylene glycol, polyvinylpyrrolidone, Ficoll (available from Amersham Pharmacia Biotech K. K.) and polysaccharide such as dextran, and sucrose are preferable. As the cell-permeating vitrificating agent, glycerol, propylene glycol, ethylene glycol and DMSO are preferable. In addition, as the solvent, physiological salt solutions are preferable as well as in cases of the slow freezing method and the rapid freezing method. More concretely, there are DMEM, phosphate buffer, Ringer's solution, Ringer-Locke's solution, Tyrode's salt solution, Earle's solution, Hanks' solution, Locke's solution, Eagle's minimum essential medium, Ham's synthetic medium F12, Green's medium, Leibovitz's L-15 medium, Cheese essential medium, modified Eagle's medium, Waymouth's medium, Kreb's medium, as well as serum-free medium MCDB153, MCDB151, MCDB104, MCDB131, MCDB402, MCDB201, MCDB302, MCDB105 and MCDB110.

"Matrix" used in the present invention must be a material excellent in the biocompatibility so that it is not rejected by the immune function of a patient when the tissue equivalent is applied to the patient. In addition, since the matrix does not need to be removed later, the matrix is preferably a biodegradable material. More particularly, collagen, gelatin, chitin, chitosan, fibrin, as well as mucopolysaccharides such as chondroitin, chondroitin sulfate and hyaluronic acid which are a tissue derived biomaterial; polyglycolic acid, polylactic acid and a mixture thereof which are a biodegradable polymer; materials which have the better cell adherability among non-biodegradable synthetic polymers such as polyurethane, polystyrene, polyacrylate; and a mixture thereof are preferable. Further, atelocollagen, collagen, gelatin, chondroitin sulfate and hyarulonic acid are more preferable, because they are excellent in the biocompatibility and have porous and sponge-like form so that cells are easily held in the matrix. It is desirable that the pore is vertically directional pores to a cell inoculating plane so that cells are easily permeated into the sponge upon inoculating of the cells, and the pores having a constant size are formed on the surface and/or plane opposite to the surface. A pore size is usually preferable in a range of 20 µm to 1000 µm in order to make the cells permeate easily upon inoculating of the cells and make an air bubble enter with difficulty. As long as an object of the present invention can be attained, the numerical ranges are not limited them.

"Tissue equivalent" in the present invention means a structure retaining the function equivalent to at least one a part of a mammal tissue, which was made by combining a matrix and mammal-derived cells. Therefore, the tissue equivalent in the present invention contains the matrix and the cells representative in the tissue. Since these representative cells have the activity equivalent to those of cells of the tissue on or in the interior of the matrix, the tissue equivalent can be used (1) for the purpose of transplantation or embedding by a variety of methods in order to substitute whole or a part of a tissue or an organ of a patient in which the tissue or the organ becomes dysfunctional due to damage or excision of the tissue or (2) for the purpose of investigating interaction between various products or raw materials and a tissue, or the effect of them on a tissue.

"Artificial skin" in the present invention means a mammal (in particular, human) skin tissue equivalent made by combining a matrix and a mammal skin-derived cell (fibroblast, epidermal cell, vascular endothelial cell, Langerhans' cell, melanocyte, adipocyte, hair matrix cell and hair papilla cell and the like). The artificial skin in the present invention is applied to various skin defect parts such as burn site, ulcer site, decubitus and donor site for the purpose of promoting wound healing. Various cells which constitute the artificial skin grow at a wound surface and, at the same time, synthesize and secrete a substance effective for wound healing (cytokines which promote wound healing by promoting growth and migration of various cells and secretion of growth factors, extracellular substrates which serve as a scaffold when surrounding cells migrate and reconstruct tissues).

"Artificial liver" in the present invention means a mammal liver tissue equivalent made by combining a matrix and mammal-derived hepatocyte. The artificial liver was developed for the purpose of substituting a part of the function responsible by liver such as metabolism and synthesis of substance essential for life, catabolism of harmful substance, and removal of intermediate metabolites, and clinical applications thereof are expected as a method for a treating patient with terminal acute and chronic hepatic insufficient.

According to the general method for making an artificial skin, a cell suspension is prepared using a skin-derived cell and, thereafter, the cell suspension is inoculated on a matrix to obtain the artificial skin. Preparation of an artificial skin from fibroblast is exemplified below.

First, a skin (including epidermis and a part or a whole layer of dermis) taken under the clean environment is disinfected and soaked in a physiological saline or Hanks' solution containing an antibiotic. This skin is soaked in DMEM in which the dispase concentration is adjusted to 1000 IU/ml and, thereafter, the skin is separated into dermis and epidermis. The resulting dermis is cut into small pieces with scissors, ground using a homogenizer, soaked in 0.5% collagenase solution (DMEM containing 0.5% (w/v) collagenase), and shaken at 37°C to dissolve a connective tissue. Then, centrifugation is performed at about 200 to 1000×g to collect dermis fibroblasts. The resulting fibroblasts are cultured at 37°C in DMEM in which FBS (bovine fetal serum) is added to 10% (v/v) (hereafter, refers to DMEM + 10% FBS) or the like as a culturing solution. If necessary, fibroblasts are subcultured. The cultured fibroblasts are peeled from a culturing flask using 0.25% trypsin solution (phosphate buffer containing 0.25% (w/v) trypsin and 0.005 mM sodium ethylene diamine tetraacetate) and centrifuged to collect it. The resulting precipitate of fibroblasts is suspended with DMEM and the like to prepare a suspension of fibroblasts. The cell concentration of the resulting fibroblast suspension is measured using a Bürker-Türk hemocytometer. Then, fibroblasts are collected by performing centrifugation again and a suspension is prepared to the density of 1×10⁴ to 5×10⁶ cells/ml, preferably 5×10⁵ to 2×10⁶ cells/ml using a cryopreserving solution containing a cryoprotectant such as glycerol and the like. The above-mentioned fibroblast suspension is inoculated on a collagen sponge having vertically directional pores obtained by gelling and lyophilizing a solution of porcine or bovine-derived aterrocollagen, at a cell density of 1×10² to 1×10⁶ cells/cm², preferably 1×10⁴ to 2×10⁵ cells/cm². After allowing to stand so as to permeate the cell suspension into the sponge, a tissue equivalent is obtained.

In the case of an artificial liver, as a method for isolating a cell, there is a collagenase perfusion method of perfusing a collagenase solution into a hepatic tissue to disperse cells but the present invention is not limited to such isolating technology. In addition, as a medium used, there are DMEM, Cheese essential medium, modified Eagle's medium, Leibovitz's medium, Waymouth's medium, Kreb's medium, Green's medium and L-15 medium in which 10% (v/v) FBS is added thereto. Concretely, Green's medium in which 10% (v/v) FBS, 10ng/ml human FGF and 13µg/ml heparin sodium is added thereto is preferable.

In the case of an artificial cornea, as an example of a method for isolating corneal epithelial, keratocyte and corneal endothelial cells, there is known a method for peeling a corneal epithelial cell by soaking a cornea into dispase solution and warming and shaking, then isolating the corneal epithelial cells by peeling a corneal epithelium from a cornea and cutting into small pieces under a microscope using a blade of scissors or a razor, placing the resulting cornea from which the corneal endothelial cells and the corneal epithelial cells are removed, on a dish to culture it, and collecting cells running from a tissue to isolate keratocyte (Adclheid I. Schneider et al., In Vitro Cell. Dev. Biol-Animal, Vol.35. 515-526 (1999) and Yoichi Minami et al., Investigative Ophthalmology & Visual Science, Vol.34. 2316-2324 (1993)), but the present invention is not limited to this isolating technology. In addition, as a medium used, in the case of keratocyte, there are DMEM, modified Eagle's medium, Eagle's minimum essential medium, and modified Eagle's medium in which 10% (v/v) FBS is added thereto, DMEM + 10% FBS is preferable. In the case of corneal epithelial cells, there is a medium in which 10% (v/v) FBS is added to Green's medium and the like.

While the present invention is explained through examples below, the present invention is not limited thereto.

### EXAMPLE 1-1

Slow cooling cryopreservation of artificial skin derived from human fibroblast

### A. Cryopreservation of artificial skin according to the method of the present invention

Human fibroblasts were cultured in DMEM + 10% FBS using a culturing flask (culturing area 80cm²). The fibroblasts in the logarithmic growth phase were treated with 0.25% trypsin solution to collect them. Cells were suspended in three kinds of cryopreserving solutions of FBScryo (DMEM containing 20% (v/v) FBS and 10% (v/v) grycerol), Cell Banker II (available from Nippon Zenyaku Kogyo Co., Ltd.)) and Cellvation (available from ICN Biomedicals Inc.)), respectively, to prepare cell suspensions of 9.0×10⁵ cells/ml. Each 0.5 ml of cell suspension was inoculated on a circular collagen sponge having a diameter of 22mm which had been placed in a 12-well plate in advance (1.2×10⁵ cells/cm²). After allowing to stand so as to permeate the cell suspension into the sponge, the 12-well plate was cooled at a rate of -0.5 to -2°C/min. to freeze. A-1) Thawing of the artificial skin and measurement of the viability

After preserved at -80°C for a necessary period of time, the artificial skin was allowed to stand under the conditions of 5% carbonic acid gas and 37°C for 10 to 15 minutes to thaw. After the medium was aspirated to remove, the artificial skin was washed with 2 ml of DMEM + 10% FBS or DMEM twice. After 2 ml of DMEM + 10% FBS or DMEM was added, culturing was performed under the conditions of 5% carbonic acid gas and 37°C for 15 hours or longer. Then, the resulting artificial skin was soaked in 5.0 ml of 0.5% collagenase solution, and shaken in a water bath at 37°C for 5 to 10 minutes to dissolve the tissue equivalent. Cells were collected by the centrifugation procedures for 5 minutes under the conditions of about 400×g and 4°C, and the viability was measured. The viability of the cells is shown in Table 1. In the case using either of cryopreserving solutions, it was shown that the viability is rapidly lowered at freezing and, thereafter, the viability is hardly changed or gradually lowered by about 1 month. In addition, the viability of 50% or greater was obtained even after 1 month.

**TABLE 1**

| Cryopreserving solution | Viability of cells (%) | | | | |
|---|---|---|---|---|---|
| | 0 day | 1 day | 1 week | 1 month | 3 months |
| FBScryo | 97.2 | 70.2 | 62.9 | 58.2 | 42.3 |
| Cellbanker II | 97.2 | - | 59.8 | 67.3 | 39.5 |
| Cellvation | 97.2 | - | 69.9 | 59.0 | 57.1 |

### A-2) Thawing of artificial skin and measurement of biological activity

### A-2-1: Cell growth rate

The artificial skin preserved at -80°C was allowed to stand under the conditions of 5% carbonic acid gas and 37°C for 10 to 15 minutes to thaw. After the medium was aspirated to remove, the artificial skin was washed with 2 ml of DMEM + 10% FBS or DMEM twice. After 2 ml of DMEM + 10% FBS or DMEM was added, culturing was performed under the conditions of 5% carbonic acid gas and 37°C for 3 days or 7 days. Then, the resulting artificial skin was soaked in 5.0 ml of a 0.5% collagenase solution and shaken in a water bath at 37°C for 5 to 10 minutes to dissolve the artificial skin. Cells were collected by the centrifugation procedures for 5 minutes under the conditions of about 400×g and 4°C, the total number of cells, the number of living cells and the viability of cells were counted by trypan blue dye exclusion method. In particular, in the case where cultured in DMEM + 10% FBS, the number of living cells was increased by 1.4-fold over after 3 day culture to after 7 day culture. The number of living cells in the artificial skin after thawing is shown in Table 2.

**TABLE 2**

| Preservation method | Cells | Preservation medium | Culture medium | The number of living cells (cells) | |
|---|---|---|---|---|---|
| | | | | After 3 day culture | After 7day culture |
| The present invention | | | | | |
| Slow Freezing | Suspend | FBScryo | DMEM+10%FBS | 1.41×10⁵ | 1.94×10⁵ |
| Slow Freezing | Suspend | FBScryo | DMEM | 1.30×10⁵ | 1.42×10⁵ |

| Comparative Example | | | | | |
|---|---|---|---|---|---|
| Nonfreezing | Adhesion | DMEM+10%FBS | DMEM+10%FBS | 3.07×10⁵ | 2.44×10⁵ |
| Nonfreezing | Adhesion | DMEM | DMEM | 2.36×10⁵ | 2.04×10⁵ |

### A-2-2: Vascular endothelial growth factor (VEGF) producing ability

The artificial skin preserved at -80°C was allowed to stand under the conditions of 5% carbonic acid gas and 37°C for 10 to 15 minutes to thaw. After the medium was aspirated to remove, the artificial skin was washed with 2 ml of DMEM + 10% FBS or DMEM twice. After each 2 ml of medium was respectively added, culturing was performed under the conditions of 5% carbonic acid gas and 37°C. The culture supernatant was collected 3 day after culturing, and the amount of VEGF in the supernatant was measured by an enzyme immunoassay (ELISA). As a control, the similar measurement was performed on an artificial skin which had not been frozen. In the amount of produced VEGF per a living cell, there was recognized a little difference between the frozen artificial skin (1) and 2) in Table 3) and the non-frozen artificial skin (3) and 4) in Table 3). The amount of VEGF (pg/ml/10⁵ living cells) produced by the artificial skin after 3 day culture is shown in Table 3.

**TABLE 3**

| | Preservation method | Culture medium | The amount of produced VEGF (pg/ml/10⁵ living cells) |
|---|---|---|---|
| The present invention | 1) Slow freezing | DMEM+10%FBS | 1399 |
| | 2) Slow freezing | DMEM | 1474 |
| Comparative Example | 3) Nonfreezing | DMEM+10%FBS | 983 |
| | 4) Nonfreezing | DMEM | 1137 |

### A-2-3: Collagen type I producing ability

The artificial skin preserved at -80°C was allowed to stand under the conditions of 5% carbonic acid gas and 37°C for 10 to 15 minutes to thaw. After the medium was aspirated to remove, the artificial skin was washed with 2 ml of DMEM twice. After 2 ml of DMEM was added, culturing was performed under the conditions of 5% carbonic acid gas and 37°C. The culture supernatant was collected after 3 day culture, and the amount of collagen type I in the supernatant was measured by ELISA method. As a control, the similar measurement was performed on the artificial skin which had not been frozen. Since an anti-collagen type I antibody used by the present inventors cross-reacts with bovine collagen in serum contained in the medium, the quantitation of collagen type I was performed only on the artificial skin which had been cultured in DMEM. The amount of produced collagen per a living cell after 3 day culture was almost the same as that of the artificial skin which had not been frozen. The amount of produced collagen type I (ng/ml/105 living cells) of an artificial skin after 3 day culture is shown in Table 4.

**TABLE 4**

| Preservation method | Culture medium | The amount of produced collagen type I (ng/ml/10⁵ living cells) |
|---|---|---|
| Slow freezing (The present invention) | DMEM | 1883 |
| Nonfreezing (Comparative Example) | DMEM | 1928 |

### B. Cryopreservation of artificial skin by the conventional method

Human fibroblasts were cultured in DMEM10% + FBS using a culturing flask (culturing area 80 cm²). The fibroblasts in the logarithmic growth phase were treated with 0.25% trypsin solution to collect it. Cells were suspended in DMEM10% + FBS to prepare cell suspension of 9.0×10⁵ cells/ml. Each 0.5 ml of cell suspension was inoculated on a circular collagen sponge having a diameter of 22mm which had been placed in a 12-well plate in advance (1.2×10⁵ cells/cm²). The 12-well plate was cultured under the conditions of 5% carbonic acid gas and 37°C for 15 hours or longer. After adherence of cells was observed, the medium was aspirated to remove, and cells were washed with 2 ml of the cryopreserving solution (FBScryo, Cell Banker II, Cellvation) twice. To each well was added 2 ml of a cryopreserving solution, the 12-well plate was cooled at a rate of -0.5 to -2°C/min. to freeze.

### B-1) Thawing of artificial skin and measurement of viability

After preserved at -80°C for a necessary period of time, the artificial skin was allowed to stand under the conditions of 5% carbonic acid gas and 37°C for 10 to 15 minutes to thaw. After the medium was aspirated to remove, the artificial skin was washed with 2 ml of DMEM + 10% FBS or DMEM twice. After 2 ml of DMEM + 10% FBS or DMEM was added, culturing was performed under the conditions of 5% carbonic acid gas and 37°C for 15 hours or longer. Then, the resulting artificial skin was soaked in 5.0 ml of a 0.5% collagenase solution, and shaken in a water bath at 37°C for 5 to 10 minutes to dissolve the tissue equivalent. Cells were collected by the centrifugation procedures for 5 minutes under the conditions of about 400×g and 4°C. The artificial skin was thawed at 1 day after freezing, and the total number of cells, the number of living cells and the viability of cells were measured. When cells were gradually cooled and frozen in the state where they adhered to the collagen sponge, the viability of cells were as very low as 10 to 20% irrespective of a kind of the cryopreserving solution. The viability of cells is shown in Table 5.

**TABLE 5**

| Cryopreserving solution | Composition or manufactures | Viability of cells (%) |
|---|---|---|
| FBScryo | FBS20%(v/v), Glycerol 10%(v/v), DMEM | 11 |
| Cellbanker II | Nippon Zenyaku Kogyo Co., Ltd | 15 |
| Cellvation | ICN Biomedicals, Inc. | 15 |

### C. Correlation between cell form at freezing and viability of cells

In the conventional method for freezing an artificial skin in the state where cells adhere to collagen sponge, the viability of cells was very low irrespective of a kind of a cryopreserving solution. A difference in the viability was recognized between the artificial skin according to the present method and the artificial skin frozen in the state where cells adhered to the collagen sponge, because the cell forms at freezing were different. Then, in order to reveal the relationship between the cell form at freezing of the artificial skin and the viability, the artificial skin was prepared according to the same manner as in Example 1-1 A except that a culturing time before freezing was changed, and the viability of cells after freezing and thawing were measured. More particularly, after the cell suspension was allowed to stand until it permeated into the sponge (present invention), or after culturing was performed at 37°C for 2 hours or 15 hours, the artificial skin was prepared and frozen according to the same manner as in Example 1-1 A. In addition, as a control, the viability of cells was measured similarly on the artificial skin in which cells were cultured at 37°C for 48 hours after inoculating. The viability was low in an artificial skin in which cells were cultured for 2 hours to 15 hours after inoculating, while the very high viability was obtained in the artificial skin which was frozen immediately after inoculating of cells. When microscopic observation was performed before freezing, it was confirmed that more than half of cells adhered to the sponge in the artificial skin which was cultured for 2 hours after inoculating and all the cells adhered to the sponge in the artificial skin which had been cultured for 15 hours or longer. That is, it was considered that the viability of cells were different depending upon the cell form at freezing. A cell recovery rate of the artificial skin which had been frozen in the state where no cell adhered, was 90% or more as compared with the artificial skin which had not been frozen. Therefore, it was considered that cells are retained in the collagen sponge even when washing procedures were performed after the artificial skin has been thawed. The viability and the total number of cells are shown in Table 6.

**TABLE 6**

| | Cryopreservation method | Preculture | Viability of cells (%) | Total cells (cells) |
|---|---|---|---|---|
| The present invention | 1) Slow freezing | Without preculture | 71.2 | 2.21×10⁵ |
| | 2) Slow freezing | 2 hours | 20.1 | 2.04×10⁵ |
| Comparative Example | 3) Slow freezing | 15 hours | 19.8 | 2.51×10⁵ |
| | 4) Nonfreezing | 48 hours | 90.7 | 2.31×10⁵ |

### EXAMPLE 1-2

### Rapid cryopreservation of artificial skin derived from human fibroblast

### A. Cryopreservation of artificial skin according to the method of the present invention

Human fibroblast was cultured in DMEM + 10% FBS using a culturing flask (culturing area 80cm²). The fibroblast in the logarithmic growth phase was treated with 0.25% trypsin solution to collect it. Cells were suspended in FBScryo to prepare a cell suspension of 9.0×10⁵ cells/ml. Each 0.5 ml of the cell suspension was inoculated on a circular collagen sponge having a diameter of 22mm which had been placed in a 12-well plate in advance (1.2×10⁵ cells/cm²). After allowing to stand so as to permeate the cell suspension into the sponge, the 12-well plate was sealed with plastic tape. The 12-well plate was frozen and preserved by directly putting into ultra-deep freezer of 152°C.

### A-1) Thawing of artificial skin and measurement of viability

The artificial skin preserved at -152°C 1 day after freezing was allowed to stand under the conditions of 5% carbonic acid gas and 37°C for 3 minutes to thaw. After the medium was aspirated to remove, the artificial skin was washed with 2 ml of DMEM + 10% FBS twice. After 2 ml of DMEM + 10% FBS was added, culturing was performed under the conditions of 5% carbonic acid gas and 37°C for 15 hours or longer. Then, the resulting artificial skin was soaked in 5.0 ml of 0.5% collagenase solution, and shaken in a water bath at 37°C for 5 to 10 minutes to dissolve the tissue equivalent. Cells were collected by the centrifugation procedures for 5 minutes under the conditions of about 400×g and 4°C, the total number of cells, the number of living cells and the viability of cells were counted by trypan blue dye exclusion method. As a control, the viability was similarly measured on the artificial skin which had not been frozen. The viability that is as high as 77.4% was obtained in the artificial skin which had been rapidly frozen at -152°C. This result can be said to be equivalent to that of slow freezing. In addition, adherence of cells to the collagen sponge by extension of a pseudopodium was confirmed by thawing an artificial skin which had been frozen according to the present method and, thereafter, culturing overnight. The viability of cells is shown in Table 7.

**TABLE 7**

| Preculture | Medium on inoculating cells | Cryopreserving solution | Freezing temperature (°C) | Viability of cells (%) |
|---|---|---|---|---|
| Freezing (The present invention) | | | | |
| Without preculture | FBSCryo | FBScryo | -152°C | 77.4 |

| Nonfreezing (Comparative Example) | | | | |
|---|---|---|---|---|
| 48 hours | DMEM+10%FBS | Nonfreezing | - | 88.7 |

### EXAMPLE 2

### Cryopreservation of artificial skin derived from epidermal cell

### A. Method of Preparation and cryopreservation of artificial skin according to the method of the present invention

Human epidermal cells were cultured in the Green medium containing 3% (v/v) FBS (hereafter refers to Green medium + 3% FBS) using a culturing flask (culturing area 80 cm²). The human epidermal cells were treated with 2 ml of a PBS (-) solution (phosphate buffer) which had been adjusted to 1000 unit/ml dispase (available from Godo shusei Co., Ltd.) and collected. The collected epidermal cells were further treated with 0.25% trypsin solution to make into single cells. The cells were suspended in a cryopreserving solution A (Green medium containing 10% (v/v) grycerol and 20% (v/v) FBS) to prepare a cell suspension of 2.5×10⁶ cells/ml. Each 0.5 ml of the suspension was inoculated on a circular collagen sponge having a diameter of 22 mm which had been placed in a 12-well plate in advance (3.3×10⁵ cells/cm²). Then, the artificial skin was prepared according to the following method.
1) After inoculating, cells were allowed to stand to sufficiently permeate into the sponge, and cooled to -80°C at a rate of -0.2 to -5°C/min. to freeze (slow freezing method).
2) After inoculating, cells were allowed to stand to sufficiently permeate into the sponge, and placed in ultra-deep freezer at -80°C to freeze (rapid freezing method).
3) After inoculating, cells were allowed to stand to sufficiently permeate into a sponge, and placed in ultra-deep freezer at -152°C to freeze (rapid freezing method).

### B. Preparation method of artificial skin according to the conventional method and artificial skin as control

Human epidermal cells were cultured in the Green medium + 3% FBS using a culturing flask (culturing area 80 cm²). The human epidermal cells were treated with 2 ml of PBS (-) solution (phosphate buffer) which had been adjusted to 1000 unit/ml dispase (available from Godo shusei Co., Ltd.), and collected. The collected epidermal cells were further treated with a 0.25% trypsin solution to make into single cells. The cells were suspended in the Green medium + 3% FBS to prepare a cell suspension of 2.5×10⁶ cells/ml. Each 0.5 ml of the suspension was inoculated on a circular collagen sponge having a diameter of 22 mm which had been placed in a 12-well plate in advance (3.3×10⁵ cells/cm²).

Then, the artificial skin was prepared according to the following method.
4) After inoculating, culturing was performed overnight (15 hours or longer) under the conditions of 5% carbonic acid gas and 37°C. After the medium was aspirated to remove, the cells were washed with cryopreserving solution A twice. Each 1.5 ml of the cryopreserving solution A was added to each well, which was cooled to -80°C at a rate of -0.2 to 5°C/min. to freeze (slow freezing method).
5) The culturing was performed for 2 days without freezing, and the viability of cells was measured according to the method of C (described below).

### C. Thawing of artificial skin and measurement of viability

The viabilitys of cells in artificial skins prepared in the above mentioned Example 2 A and B were measured according to the following method.

The artificial skin which had been cryopreserved in the ultra-deep freezer at -180°C or -152°C for 1 to 7 days, was soaked in a water bath at 37°C for 5 to 10 minutes to thaw. After the medium was aspirated to remove, the artificial skin was washed with 2.0 ml of Green medium + 3% FBS twice. Thereto 1.5ml of the same medium was added, followed by culturing overnight (15 hours or longer) under the conditions of 5% carbonic acid gas and 37°C. Then, the resulting artificial skin was soaked in 5.0 ml of 0.5% collagenase solution, and shaken in the water bath at 37°C for 5 to 10 minutes to dissolve the collagen sponge. Cells were collected by the centrifugation procedures of about 400×g, 4°C and 5 minutes. After the supernatant was removed, 3.0 ml of 0.25% trypsin solution was added to cells, which was shaken in the water bath at 37°C for 5 to 10 minutes. Again, cells were collected by the centrifugation procedures of about 400×g, 4°C and 5 minutes. Cells were suspended in the Green medium + 3% FBS, and the viability of cells was measured by trypan blue dye exclusion method.

As a result, the high viability was obtained in the artificial skin in which cells were inoculated on the collagen sponge and, thereafter, slowly cooled and frozen (the present invention, 1) in Table 8) or rapidly frozen (the present invention, 2) and 3) in Table 8) before cells adhered. To the contrary, in the artificial skin in which, cells were inoculated and, thereafter, slowly cooled and frozen after culturing for 15 hours (conventional method, 4) in Table 8), the viability of cells was low. The viability of cells is shown in Table 8.

**TABLE 8**

| | Preservation method | Preculture | Freezing temperature | Viability of cells (%) |
|---|---|---|---|---|
| The present invention | 1) Slow freezing | Without preculture | -80 | 84.6 |
| | 2) Rapid freezing | Without preculture | -80 | 74.6 |
| | 3) Rapid freezing | Without Preculture | -152 | 83.2 |
| Comparative Example | 4) Slow freezing | 15 hours | -80 | 19.1 |
| | 5) Nonfreezing | 48 hours | - | 89.6 |

### EXAMPLE 3

### Cryopreservation of artificial blood vessel derived from vascular endothelial cell

### A. Method of Preparation and cryopreservation of artificial blood vessel according to method of the present invention.

Human vascular endothelial cells were cultured in the Green medium containing 10% (v/v) FBS and 10ng/ml human FGF (human fibroblast growth factor) using a culturing flask (culturing area 80 cm²). The human vascular endothelial cells were treated with 0.25% trypsin solution to collect. The cells were suspended in the cryopreserving solution B (Green medium containing 10% (v/v) grycerol, 20% (v/v) FBS and 10ng/ml human FGF) to prepare a cell suspension of 1.4×10⁶ cells/ml. Each 0.6 ml of the suspension was inoculated on the circular collagen sponge having a diameter of 22 mm which had been placed in a 12-well plate in advance (2.2×10⁵ cells/cm²).

Then, the artificial blood vessel was prepared according to the following method.
1) After inoculating, cells were allowed to stand to sufficiently permeate into the sponge, and cooled to -80°C at a rate of -0.2 to -5°C/min. to freeze (slow freezing method).

### B. Preparation method of artificial blood vessel according to the conventional method and artificial blood vessel as control

Human vascular endothelial cells were cultured in the Green medium containing 10% (v/v) FBS and 10ng/ml human FGF (human fibroblast growth factor) using a culturing flask (culturing area 80 cm²). The human vascular endothelial cells were treated with 0.25% trypsin solution to collect. The cells were suspended in the Green medium containing 10% (v/v) FBS and 10ng/ml human FGF to prepare a cell suspension of 1.4×10⁶ cells/ml. Each 0.6 ml of the suspension was inoculated on the circular collagen sponge having a diameter of 22 mm which had been placed in a 12-well plate in advance (2.2×10⁵ cells/cm²).

Then, the artificial blood vessel was prepared according to the following method.
2) After inoculating, the cells were cultured overnight and washed with the cryopreserving solution B twice and, thereafter, cooled to -80°C at a rate of -0.2 to -5°C/min. to freeze (slow freezing method).
3) The culturing was performed for 2 days without freezing and the viability of cells was measured according to the method of C (described below).

### C. Thawing of artificial blood vessel and measurement of viability

The viability of cells in the artificial blood vessel prepared in the above mentioned Example 3 A and B were measured according to the following method.

The artificial blood vessel which had been cryopreserved in the ultra-deep freezer at -80°C for 1 to 7 days, was soaked in a water bath at 37°C for 5 to 10 minutes to thaw. After the medium was aspirated to remove, the artificial blood vessel was washed with 2.0 ml of Green medium containing 10% (v/v) FBS and 10ng/ml human FGF twice. Thereto 1.5ml of the same medium was added, followed by culturing overnight (15 hours or longer) under the conditions of 5% carbonic acid gas and 37°C. Then, the resulting artificial blood vessel was soaked in 5.0 ml of 0.5% collagenase solution, and shaken in the water bath at 37°C for 5 to 10 minutes to dissolve the collagen sponge. Cells were collected by the centrifugation procedures of about 400×g, 4°C and 5 minutes. After the supernatant was removed, 3.0 ml of 0.25% trypsin solution was added to cells, which was shaken in the water bath at 37°C for 5 to 10 minutes. Again, cells were collected by the centrifugation procedures of about 400×g, 4°C and 5 minutes. Cells were suspended in the Green medium containing 10% (v/v) FBS and 10ng/ml human FGF, and the viability of the cells was measured by trypan blue dye exclusion method.

As a result, the high viability was obtained in the artificial blood vessel in which cells were inoculated on the collagen sponge and, thereafter, slowly cooled and frozen before cells adhered (the present invention, 1) in Table 9). To the contrary, in the artificial blood vessel in which, cells were inoculated and, thereafter, slowly cooled and frozen after culturing for 15 hours (conventional method, 2) in Table 9), the viability of the cells was low. The viability of cells is shown in Table 9.

**TABLE 9**

| | Preservation method | Preculture | Freezing temperature (°C) | Viability of cells (%) |
|---|---|---|---|---|
| The present invention | 1) Slow freeing | Without preculture | -80 | 83.7 |
| Comparative Example | 2) Slow freezing | 15 hours | -80 | 51.1 |
| | 3) Nonfreezing | 48 hours | - | 87.7 |

### EXAMPLE 4

### Cryopreservation of artificial liver derived from hepatocyte

### A. Method of Preparation and cryopreservation of artificial liver according to the method of the present invention

Human hepatocytes were cultured in the Green medium containing 10% (v/v) FBS, 10ng/ml human FGF and 13µg/ml heparin sodium using a culturing flask (culturing area 80 cm²). The human hepatocytes were treated with 0.25% trypsin solution to collect. The cells were suspended in a cryopreserving solution C (Green medium containing 10% (v/v) grycerol, 20% (v/v) FBS, 10ng/ml human FGF and 13µg/ml heparin sodium) to prepare a cell suspension of 1.5×10⁶ cells/ml. Each 0.5 ml of the suspension was inoculated on a circular collagen sponge having a diameter of 22 mm which had been placed in a 12-well plate in advance (2.0×10⁵ cells/cm²). Then, the artificial liver was prepared according to the following method.
1) After inoculating, cells were allowed to stand to sufficiently permeate into the sponge, and cooled to -80°C at a rate of -0.2 to -5°C/min. to freeze (slow freezing method).
2) After inoculating, cells were allowed to stand to sufficiently permeate into the sponge, and placed in ultra-deep freezer at -80°C to freeze (rapid freezing method).
3) After inoculating, cells were allowed to stand to sufficiently permeate into a sponge, and placed in ultra-deep freezer at -152°C to freeze (rapid freezing method).

### B. Preparation method of artificial blood vessel as control

Human hepatocytes were cultured in the Green medium containing 10% (v/v) FBS, 10ng/ml human FGF and 13µg/ml heparin sodium using a culturing flask (culturing area 80 cm²). The human hepatocytes were treated with 0.25% trypsin solution to collect. The cells were suspended in Green medium containing 10% (v/v) FBS, 10ng/ml human FGF and 13µg/ml heparin sodium to prepare a cell suspension of 1.5×10⁶ cells/ml. Each 0.5 ml of the suspension was inoculated on a circular collagen sponge having a diameter of 22 mm which had been placed in a 12-well plate in advance (2.0×10⁵ cells/cm²).

Then, the artificial liver was prepared according to the following method.
4) After inoculating, culturing was performed overnight (15 hours or longer) under the conditions of 5% carbonic acid gas and 37°C. After the medium was aspirated to remove, the cell was washed with cryopreserving solution C twice. Each 1.5 ml of the cryopreserving solution C was added to each well, which was cooled to -80°C at a rate of -0.2 to 5°C/min. to freeze (slow freezing method).
5) After inoculating, culturing was performed overnight (15 hours or longer) under the conditions of 5% carbonic acid gas and 37°C. After the medium was aspirated to remove, the cells were washed with cryopreserving solution C twice. Each 1.5 ml of the cryopreserving solution C was added to each well, and it placed in ultra-deep freezer at -80°C to freeze.
6) The culturing was performed for 2 days without freezing, and the viability of cells was measured according to the method of C (described below).

### C. Thawing of artificial liver and measurement of viability

The viability of cells in the artificial liver prepared in the above mentioned Example 4 A and B were measured according to the following method.

The artificial liver which had been cryopreserved in the ultra-deep freezer at -80°C or -152°C for 1 to 7 days, was soaked in a water bath at 37°C for 5 to 10 minutes to thaw. After the medium was aspirated to remove, the artificial liver was washed with 2.0 ml of Green medium containing 10% (v/v) FBS, 10ng/ml human FGF and 13µg/ml heparin sodium twice. Thereto 1.5ml of the same medium was added, followed by culturing overnight (15 hours or longer) under the conditions of 5% carbonic acid gas and 37°C. Then, the resulting artificial liver was soaked in 5.0 ml of 0.5% collagenase solution, and shaken in the water bath at 37°C for 5 to 10 minutes to dissolve the collagen sponge. Cells were collected by the centrifugation procedures of about 400×g, 4°C and 5 minutes. Cells were suspended in the Green medium containing 10% (v/v) FBS, 10ng/ml human FGF and 13µg/ml heparin sodium, and the viability of the cells was measured by trypan blue dye exclusion method.

As a result, the high viability was obtained in the artificial liver in which cells were inoculated on the collagen sponge and, thereafter, slowly cooled and frozen (the present invention, 1) in Table 10) or rapidly frozen (the present invention, 2) and 3) in Table 10) before cells adhered. To the contrary, in the artificial liver in which, cells were inoculated and, thereafter, cooled and frozen after culturing for 15 hours (conventional method, 4) and 5) in Table 10), the viability of cells was low. The viability of cells is shown in Table 10.

**TABLE 10**

| | Preservation method | Preculture | Freezing temperature (°C) | Viability of cells (%) |
|---|---|---|---|---|
| The present invention | 1) Slow freezing | Without preculture | -80 | 88.5 |
| | 2) Rapid freezing | Without preculture | -80 | 78.5 |
| | 3) Rapid freezing | Without preculture | -152 | 79.6 |
| Comparative Example | 4) Slow freezing | 15 hours | -80 | 23.9 |
| | 5) Rapid freezing | 15 hours | -80 | 22.9 |
| | 6) Nonfreezing | 48 hours | - | 94.0 |

### EXAMPLE 5

### Cryopreservation of artificial cornea derived from keratocyte

### A. Method of Preparation and cryopreservation of artificial cornea according to the method of the present invention

Rabbit keratocyte was cultured in DMEM + 10% FBS using a culturing flask (culturing area 80 cm²). The rabbit keratocytes were treated with 0.25% trypsin solution to collect. The cells were suspended in the cryopreserving solution FBScryo to prepare a cell suspension of 1.0×10⁶ cells/ml. Each 0.5 ml of the suspension was inoculated on a circular collagen sponge having a diameter of 22 mm which had been placed in a 12-well plate in advance (1.3×10⁵ cells/cm²). Then, the artificial cornea was prepared according to the following method.
1) After inoculating, cells were allowed to stand to sufficiently permeate into the sponge, and cooled to -80°C at a rate of -0.2 to -5°C/min. to freeze (slow freezing method).
2) After inoculating, cells were allowed to stand to sufficiently permeate into the sponge, and placed in ultra-deep freezer at -152°C to freeze (rapid freezing method).

### B. Preparation method of artificial cornea as control

Rabbit keratocytes were cultured in DMEM + 10% FBS using a culturing flask (culturing area 80 cm²). The rabbit keratocytes were treated with 0.25% trypsin solution to collect. The cells were suspended in DMEM + 10% FBS to prepare a cell suspension of 1.0×10⁶ cells/ml. Each 0.5 ml of the suspension was inoculated on a circular collagen sponge having a diameter of 22 mm which had been placed in a 12-well plate in advance (1.3×10⁵ cells/cm²).

Then, the artificial cornea was prepared according to the following method.
3) After inoculating, culturing was performed overnight (15 hours or longer) under the conditions of 5% carbonic acid gas and 37°C. After the medium was aspirated to remove, the cells were washed with FBScryo twice. Each 1.5 ml of FBScryo was added to each well, which was cooled to -80°C at a rate of -0.2 to 5°C/min. to freeze (slow freezing method).
4) After inoculating, culturing was performed overnight (15 hours or longer) under the conditions of 5% carbonic acid gas and 37°C. After the medium was aspirated to remove, the cells were washed with FBScryo twice. Each 1.5 ml of FBScryo was added to each well, and it placed in ultra-deep freezer at -80°C to freeze.
5) The culturing was performed for 2 days without freezing, and the viability of cells was measured according to the method of C (described below).

### C. Thawing of artificial cornea and measurement of viability

The viability of cells in the artificial cornea prepared in the above mentioned Example 5 A and B were measured according to the following method.

The artificial cornea which had been cryopreserved in the ultra-deep freezer at -80°C or -152°C for 1 to 7 days, was soaked in a water bath at 37°C for 5 to 10 minutes to thaw. After the medium was aspirated to remove, the artificial cornea was washed with 2.0 ml of 10% (v/v) FBS twice. Thereto 1.5ml of the same medium was added, followed by culturing overnight (15 hours or longer) under the conditions of 5% carbonic acid gas and 37°C. Then, the resulting artificial cornea was soaked in 5.0 ml of 0.5% collagenase solution, and shaken in the water bath at 37°C for 5 to 10 minutes to dissolve the collagen sponge. Cells were collected by the centrifugation procedures of about 400×g, 4°C and 5 minutes. The cells were suspended in the DMEM + 10% FBS, and the viability of the cells was measured by trypan blue dye exclusion method.

As a result, the high viability was obtained in the artificial cornea in which cells were inoculated on the collagen sponge and, thereafter, slowly cooled and frozen (the present invention, 1) in Table 11) or rapidly frozen (the present invention, 2) in Table 11) before cells adhered. To the contrary, in the artificial cornea in which, cells were inoculated and, thereafter, cooled and frozen after culturing for 15 hours (conventional method, 3) and 4) in Table 11), the viability of cells was low. The viability of cells is shown in Table 11.

**TABLE 11**

| | Preservation method | Preculture | Freezing temperature (°C) | Viability of cells (%) |
|---|---|---|---|---|
| The present invention | 1) Slow freezing | Without preculture | -80 | 63.5 |
| | 2) Rapid freezing | Without preculture | -152 | 72.4 |
| Comparative Example | 3) Slow freezing | 15 hours | -80 | 14.9 |
| | 4) Rapid freezing | 15 hours | -80 | 9.1 |
| | 5) Nonfreezing | 48 hours | - | 88.2 |

### INDUSTRIAL APPLICABILITY

Since the method for cryopreservation of the present invention has the higher viability of cells than that in the conventional method, a tissue equivalent useful for wound healing can be preserved for a longer period of time. In addition, since preculturing and washing steps before freezing can be omitted and washing procedure after thawing is simple in the present method, the present method is simpler and more inexpensive as compared with the conventional method. Further, since a tissue equivalent can be cryopreserved by a simple freezer (-85°C to -20°C) in the present method, a tissue equivalent can be preserved in more medical facilities. Moreover, since a tissue equivalent cryopreserved by the present method has no toxicity and high safety, it can be immediately used clinically.

## Claims

1. A method for cryopreservation of a tissue equivalent which comprises a step of suspending cells into a cryopreserving solution, a step of inoculating the cells on a matrix, and a step of freezing the thus obtained tissue equivalent before the cells adhere to the matrix.

2. The method of Claim 1, wherein the cell is one or more selected from the group consisting of fibroblast, epidermal cell, vascular endothelial cell, Langerhans' cell, melanocyte, adipocyte, hair matrix cell, hair papilla cell, smooth muscular cell, hepatocyte, keratocyte, corneal epithelial cell, and corneal endothelial cell which are derived from a mammal.

3. The method of Claim 1, wherein the step of freezing is carried out using slow freezing method.

4. The method of Claim 1, wherein the step of freezing is carried out using rapid freezing method.

5. The method of Claim 1, wherein the step of freezing is carried out using vitrification method.

6. The method of Claim 1, wherein the preservation temperature after freezing is at least -196°C to at most -20°C.

7. The method of Claim 1, wherein the tissue equivalent is an artificial skin, an artificial blood vessel, an artificial liver or an artificial cornea.

8. A cryopreserved tissue equivalent obtained by inoculating cells suspended in a cryopreserving solution on a matrix and freezing it before the cells adhere to the matrix.

9. The cryopreserved tissue equivalent of Claim 8, wherein the tissue equivalent is an artificial skin, an artificial blood vessel, an artificial liver or an artificial cornea.

10. The tissue equivalent of Claim 8, wherein a combination of the matrix and the cells is a sponge comprising atelocollagen and fibroblasts derived from humman.

11. The tissue equivalent of Claim 10, in which the sponge comprising atelocollagen has vertically directional pores.
